(19) Europäisches Patentamt
European Patent Office
Office européen des brevets

(11) Veröffentlichungsnummer: **0 141 204**
**B1**

(12) **EUROPÄISCHE PATENTSCHRIFT**

(45) Veröffentlichungstag der Patentschrift: **02.05.90**

(21) Anmeldenummer: **84111050.5**

(22) Anmeldetag: **17.09.84**

(51) Int. Cl.$^5$: **C 07 D 249/08,**
C 07 D 233/60,
C 07 D 233/61,
A 01 N 43/653, A 01 N 43/50

# (54) Hydroxyethyl-azol-Derivate.

(30) Priorität: **26.09.83 DE 3334779**

(43) Veröffentlichungstag der Anmeldung:
**15.05.85 Patentblatt 85/20**

(45) Bekanntmachung des Hinweises auf die Patenterteilung:
**02.05.90 Patentblatt 90/18**

(84) Benannte Vertragsstaaten:
**AT BE CH DE FR GB IT LI NL SE**

(56) Entgegenhaltungen:
**EP-A-0 085 842**
**EP-A-0 086 304**
**EP-A-0 086 917**
**EP-A-0 139 227**

(73) Patentinhaber: **BAYER AG**
**D-5090 Leverkusen 1 Bayerwerk (DE)**

(72) Erfinder: **Holmwood, Graham, Dr.**
**Krutscheider Weg 105**
**D-5600 Wuppertal 11 (DE)**
Erfinder: **Krämer, Wolfgang, Dr.**
**Am Eckbusch 39/45**
**D-5600 Wuppertal 1 (DE)**
Erfinder: **Büchel, Karl Heinz, Prof. Dr.**
**Dabringhausener Strasse 42**
**D-5093 Burscheid (DE)**
Erfinder: **Reinecke, Paul, Dr.**
**Lessingstrasse 11**
**D-5090 Leverkusen 3 (DE)**
Erfinder: **Brandes, Wilhelm, Dr.**
**Eichendorffstrasse 3**
**D-5653 Leichlingen 1 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 141 204 B1

## Beschreibung

Die vorliegende Erfindung betrifft neue Hydroxyethylazol-Derivate, mehrere Verfahren zu ihrer Herstellung und ihre Verwendung als Fungizide.

Es ist bereits bekannt geworden, daß bestimmte Hydroxyethyl-azol-Derivate, wie beispielsweise 1-(2,4-Dichlorphenoxy)-3,3-dimethyl-2-(1,2,4-triazol-1-yl-methyl)-2-butanol oder 1-(4-Chlorphenoxy)-3,3-dimethyl-2-(imidazol-1-yl)-2-butanol, fungizide Eigenschaften aufweisen (vgl. EP—A—0 040 345). Die Wirksamkeit dieser Verbindungen ist jedoch, insbesondere bei niedrigen Aufwandmengen und -konzentrationen, nicht immer ganz zufriedenstellend.

Die in der EP—A—0 86 917 aufgeführte allgemeine Formel (I) ist unter anderem auf fungizid wirksame Hydroxyethylazolyl-Derivate gerichtet, in denen eine Aldhyd-Gruppe über eine einfach durch Alkyl substituierte Methylengruppe mit dem Carbinolkohlenstofatom verbunden ist. Entsprechende Verbindungen, die eine zweifach durch Alkyl substituierte Methylenbrücke enthalten, werden jedoch nicht offenbart.

Es wurden neue Hydroxyethyl-azol-Derivate der allgemeinen Formel (I),

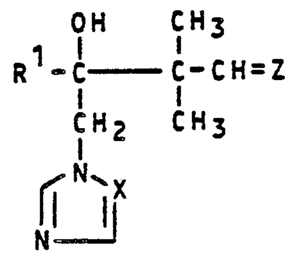

(I)

in welcher

$R^1$ für Alkyl mit 1 bis 6 Kohlenstoffatomen oder für die Gruppierung Ar—Y steht;

Ar für Naphthyl oder Phenyl steht, wobei der Phenylrest einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Nitro, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, den $—CH=NOR^2$-Rest, gegebenenfalls durch Halogen und/oder Alkyl mit 1 oder 2 Kohlenstoffatomen substituiertes Phenyl, gegebenenfalls durch Halogen und/oder Alkoxy mit 1 oder 2 Kohlenstoffatomen substituiertes Phenoxy, gegebenenfalls durch Halogen und/oder Alkyl mit 1 oder 2 Kohlenstoffatomen substituiertes Benzyl und gegebenenfalls durch Halogen und/oder Alkyl mit 1 oder 2 Kohlenstoffatomen substituiertes Benzyloxy,

X für ein Stickstoffatom oder die CH-Gruppe steht;

Y für eine direkte Bindung oder für die Gruppierungen $—CH_2—$, $—CH_2CH_2—$, $—OCH_2—$, $—SCH_2—$, —CH=CH— oder —C≡C— steht;

Z für Sauerstoff oder die $NOR^2$-Gruppe steht und

$R^2$ für Wasserstoff, für geradkettiges oder verzweigtes Alkyl mit 1 bis 6 Kohlenstoffatomen, für Alkenyl und Alkinyl mit jeweils 2 bis 6 Kohlenstoffatomen steht oder für Phenylalkyl mit 1 oder 2 Kohlenstoffatomen im Alkylteil steht, wobei der Phenylteil einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Nitro, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Benzyl und gegebenenfalls durch Halogen und/oder Alkyl mit 1 oder 2 Kohlenstoffatomen substituiertes Benzyloxy, oder

$R^2$ für gegebenenfalls einfach bis dreifach, gleich oder verschieden durch Alkyl mit 1 bis 3 Kohlenstoffatomen substituiertes Cycloalkylmethyl mit 5 oder 6 Kohlenstoffatomen im Cycloalkylteil steht,

und deren Säureadditions-Salze und Metallsalz-Komplexe gefunden.

Weiterhin wurde gefunden, daß man die Hydroxyethyl-azol-Derivate der Formel (I) sowie deren Säureadditions-Salze und Metallsalz-komplexe erhält, wenn man

a) Hydroxyethylazolyl-acetal-Derivate der Formel

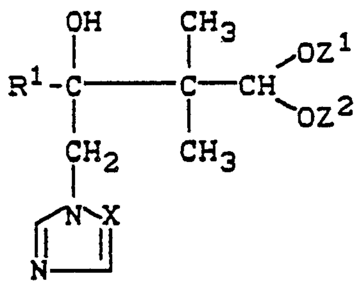

(II)

in welcher

$Z^1$ und $Z^2$ für Alkyl mit 1 bis 4 Kohlenstoffatomen stehen oder gemeinsam mit den Sauerstoffatomen, an die sie gebunden sind, für einen Dioxolan-Ring stehen und

$R^1$ und X die oben angegebene Bedeutung haben,

in Gegenwart eines Gemisches aus Wasser und einem organischen Lösungsmittel und in Gegenwart einer Säure erhitzt; und/oder

b) die nach dem Verfahren a) erhaltenen Hydroxyethyl-azol-Derivate der Formel

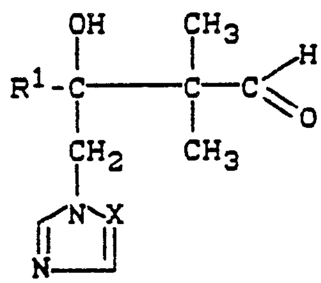

(Ia)

in welcher

$R^1$ und X die oben angegebene Bedeutung haben,

oder die Hydroxyethylazolyl-acetal-Derivate der Formel (II) mit Hydroxylamin-Derivaten der Formel

$$H_2N—O—R^2 \qquad (III)$$

in welcher

$R^2$ die oben angegebene Bedeutung hat,

in Gegenwart eines Verdünnungsmittels umsetzt; oder

c) die nach dem Verfahren b) erhaltenen Hydroxylethylazol-Derivate der Formel

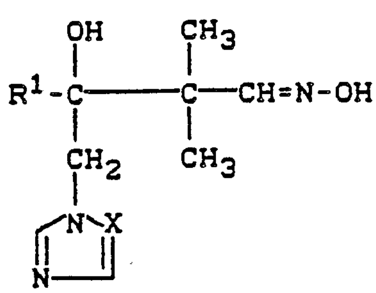

(Ib)

in welcher

$R^1$ und X die oben angegebene Bedeutung haben,

mit Halogeniden der Formel

$$Hal—R^3 \qquad (IV)$$

in welcher

Hal für Chlor, Brom oder Iod steht und

$R^3$ für die Bedeutungen von $R^2$, außer für Wasserstoff, steht,

in Gegenwart eines Verdünnungsmittels und gegebenenfalls in Gegenwart einer Base umsetzt; oder

d) Oxirane der Formel

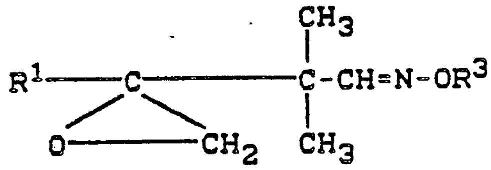

(V)

in welcher

$R^1$ und $R^3$ die oben angegebene Bedeutung haben,

mit Azolen der Formel

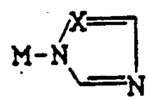

(IV)

in welcher

X die oben angegebene Bedeutung hat und

M für Wasserstoff, Natrium oder Kalium steht,

in Gegenwart eines Verdünnungsmittels und gegebenenfalls in Gegenwart einer Base umsetzt, und gegebenenfalls noch an die so erhaltenen Verbindungen der Formel (I) anschließend eine Säure oder ein Metallsalz addiert.

Die neuen Hydroxyethyl-azol-Derivate der Formel (I) weisen starke fungizide Eigenschaften auf. Dabei zeigen überraschenderweise die erfindungsgemäßen Verbindungen eine höhere Wirkung als die aus dem Stand der Technik bekannten Hydroxyethyl-azol-Derivate 1-(2,4-Dichlorphenoxy)-3,3-dimethyl-2-(1,2,4-triazol-1-yl-methyl)-2-butanol und 1-(4-Chlorphenoxy)-3,3-dimethyl-2-(imidazol-1-yl)-2-butanol, welche konstitutionell und wirkungsmäßig naheliegende Verbindungen sind. Die erfindungsgemäßen Stoffe stellen somit eine Bereicherung der Technik dar.

Die erfindungsgemäßen Hydroxyethyl-azol-Derivate sind durch die Formel (I) allgemein definiert.

Bevorzugt sind diejenigen Verbindungen der Formel (I), in denen

$R^1$ für geradkettiges Alkyl mit 1 bis 6 Kohlenstoffatomen oder für die Gruppierung Ar—Y steht;

Ar für Naphthyl oder für gegebenenfalls einfach bis dreifach, gleich oder verschieden durch Fluor, Chlor, Methyl, Trifluormethyl, Trifluormethoxy, Trifluormethylthio, Methoximinomethyl, Ethoximinomethyl, Allyloximinomethyl, sowie jeweils gegebenenfalls durch Chlor und/oder Methyl substituiertes Phenyl, Phenoxy, Benzyl oder Benzyloxy steht,

X für ein Stickstoffatom oder die CH-Gruppe steht;

Y für eine direkte Bindung oder für die Gruppierungen $—CH_2—$, $—CH_2—CH_2—$, $—OCH_2—$, $—SCH_2—$, $—CH=CH—$ oder $—C \equiv C—$ steht;

Z für Sauerstoff oder die $NOR^2$-Gruppe steht; wobei

$R^2$ für Wasserstoff, Methyl, Ethyl, n-Propyl, n-Butyl, Allyl, Propargyl, für gegebenenfalls einfach bis dreifach, gleich oder verschieden durch Fluor, Chlor, Methyl, Trifluormethyl oder Trifluormethoxy substituiertes Benzyl sowie für gegebenenfalls durch Methyl oder Ethyl substituiertes Cyclohexylmethyl steht.

Bevorzugte erfindungsgemäße Verbindungen sind auch Additionsprodukte aus Säuren und denjenigen Hydroxyethyl-azol-Derivaten der Formel (I), in denen die Substituenten $R^1$, X und Z die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Hydroxyethyl-azol-Derivaten der Formel (I), in denen die Substituenten $R^1$, X und Z die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Verbindungen der allgemeinen Formel (I) genannt:

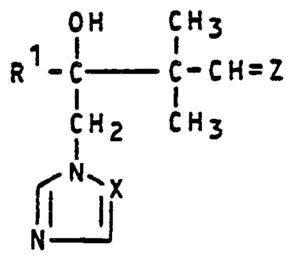

(I)

(wobei X sowohl für ein Stickstoffatom als auch für die CH-Gruppe steht):

| $R^1$ | Z |
|---|---|
| 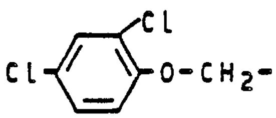 | O |
| 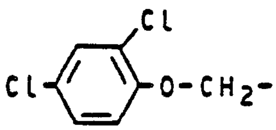 | $N-OCH_3$ |
| 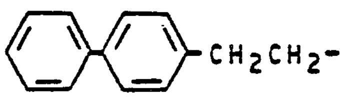 | O |
| 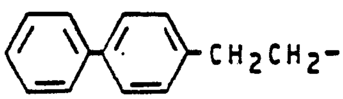 | $N-OCH_3$ |
| 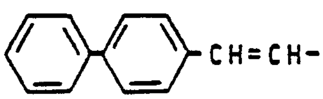 | O |
| 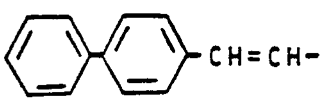 | $N-OCH_3$ |
| $CH_3$ | 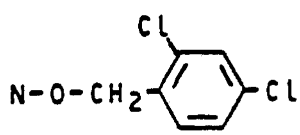 |
| $C_2H_5$ | 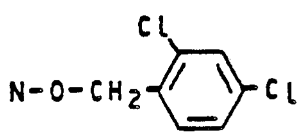 |
| $C_3H_7$ | 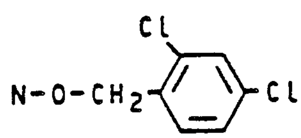 |
| $C_4H_9$ | 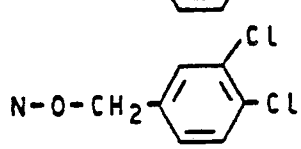 |
| $C_4H_9$ | 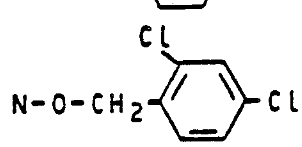 |
| $C_4H_9$ | 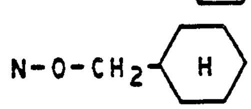 |

Fortsetzung :

| $R^1$ | Z |
|---|---|
| 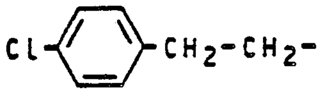 | $N-O-CH_3$ |
| 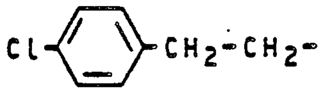 | $N-O-C_2H_5$ |
| 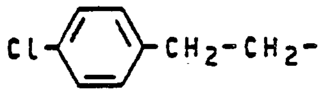 | $N-O-C_3H_7$ |
| 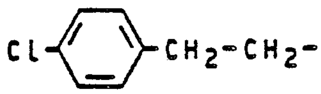 | $N-O-C_4H_9$ |
| 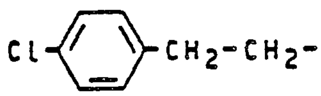 | $N-O-CH=CH_2$ |
| 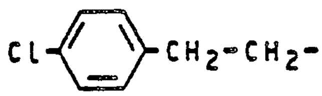 | 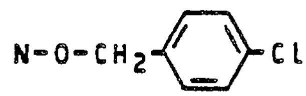 |
| 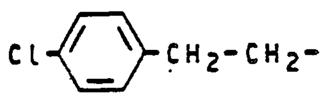 | 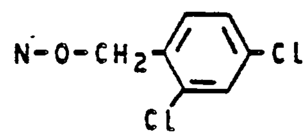 |
| 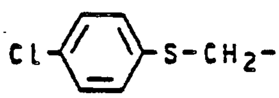 | $N-O-CH_3$ |
| 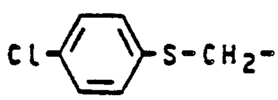 | $N-O-CH_2-CH=CH_2$ |
| 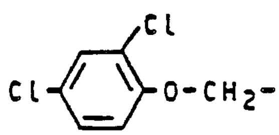 | $N-OCH_3$ |
| 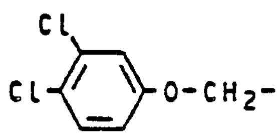 | 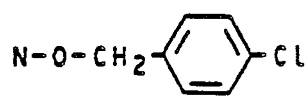 |
| 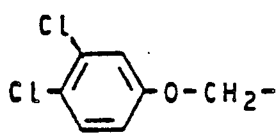 | $N-O-C_4H_9$ |
| 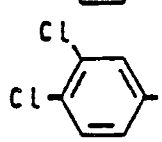 | $N-OCH_3$ |
| 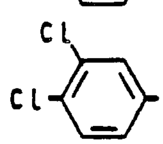 | $N-OC_2H_5$ |

Fortsetzung :

| $R^1$ | Z |
| --- | --- |
| 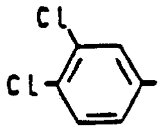 | $N-OC_3H_7$ |
| 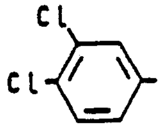 | $N-OC_4H_9$ |
| 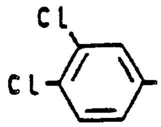 | $N-O-CH_2-CH=CH_2$ |
| 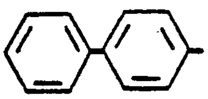 | $N-OCH_3$ |
| 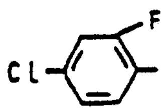 | $N-OCH_3$ |

Verwendet man beispielsweise 1-(4-Chlorphenoxy)-3-(1,3-dioxolan-2-yl)-3-methyl-2-(1,2,4-triazol-1-yl-methyl)-2-butanol in einem Ethanol/Wasser-Gemisch als Ausgangsstoff, so kann der Ablauf des erfindungsgemäßen Verfahrens (a) durch das folgende Formelschema wiedergegeben werden:

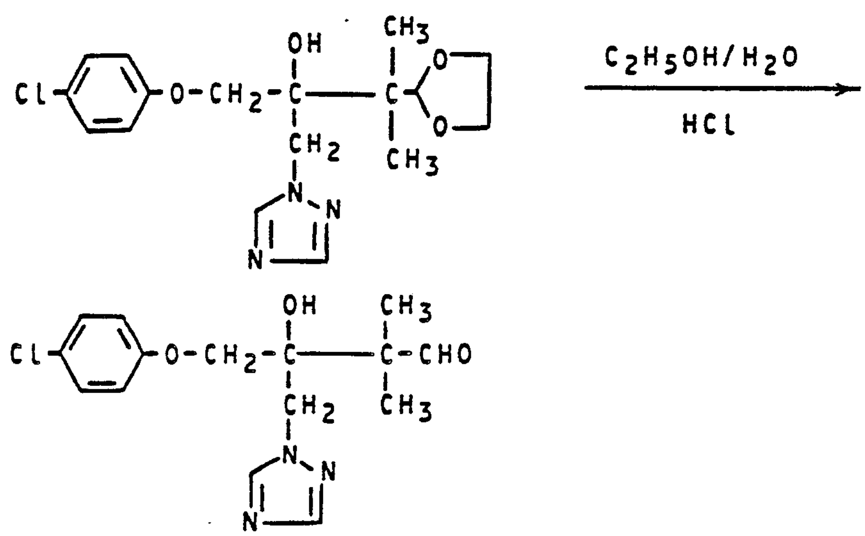


Verwendet man beispielsweise 4-(4-Chlorphenoxy)-2,2-dimethyl-3-hydroxy-3-(1,2,4-triazol-1-yl-methyl)-butanal und O-Methyl-hydroxylamin-hydrochlorid als Ausgangsstoffe, so kann der Ablauf des erfindungsgemäßen Verfahrens (b) durch das folgende Formelschema wiedergegeben werden:

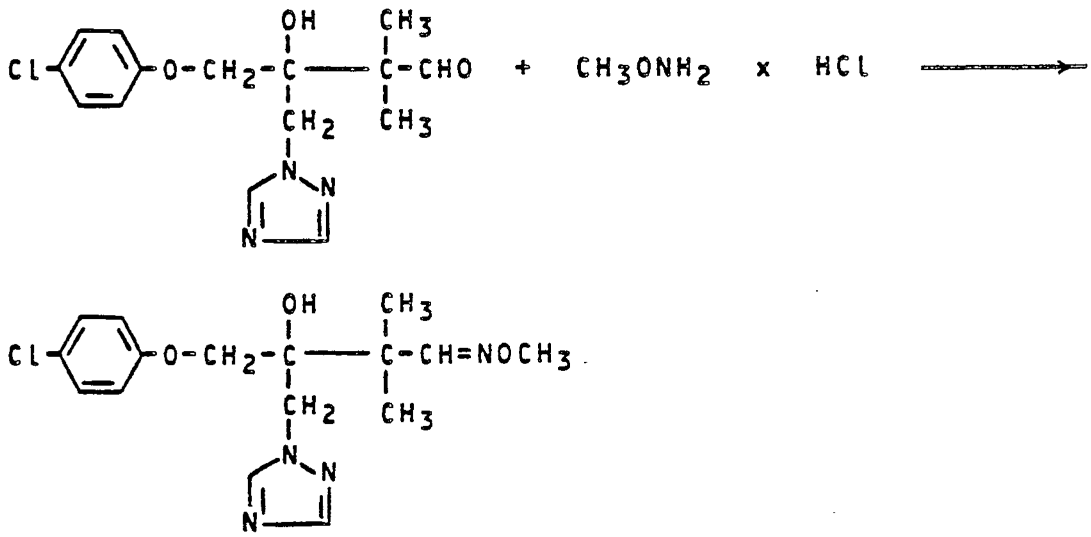

Verwendet man beispielsweise 1-(4-Chlorphenoxy)-3-hydroximino-methyl-3-methyl-2-(1,2,4-triazol-1-yl-methyl)-2-butanol und 4-Chlorbenzylchlorid als Ausgangsstoffe, so kann der Ablauf des erfindungsgemäßen Verfahrens (c) durch das folgende Formelschema wiedergegeben werden:

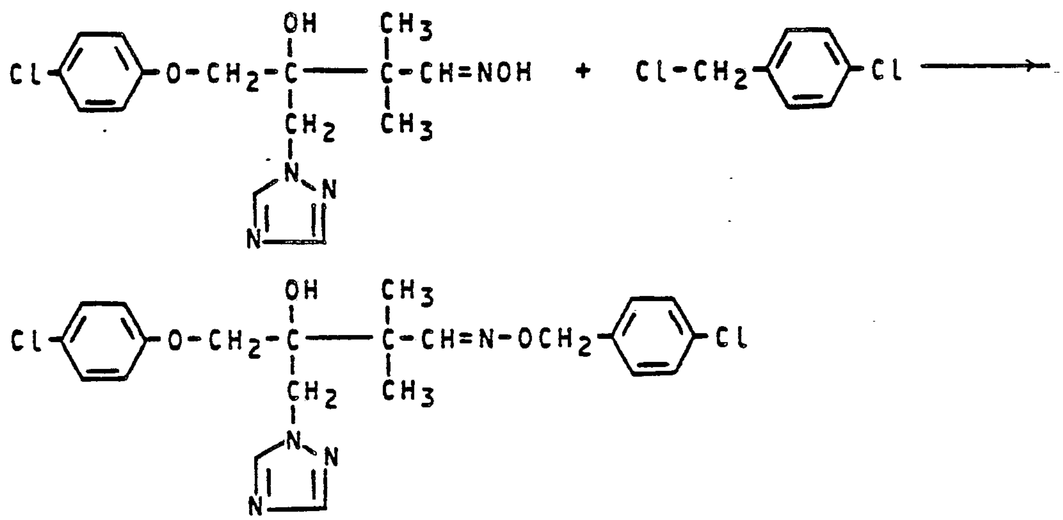

Verwendet man beispielsweise 2-(2,4-Dichlorphenyl)-2-(2-methoximinomethyl-2-propyl)-oxiran und 1,2,4-Triazol als Ausgangsstoffe, so kann der Ablauf des erfindungsgemäßen Verfahrens (d) durch das folgende Formelschema wiedergegeben werden:

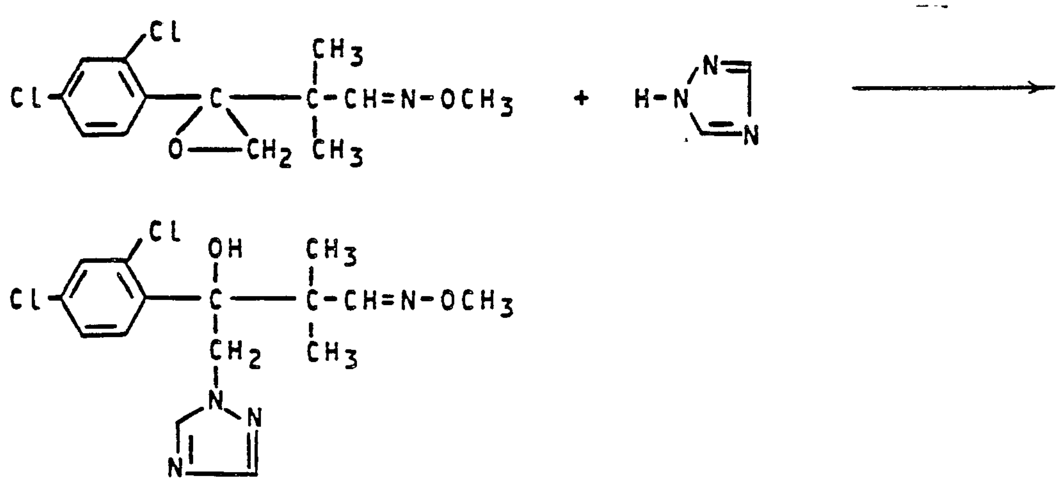

Die für die Durchführung des erfindungsgemäßen Verfahrens (a) als Ausgangsstoffe zu verwendenden Hydroxyethylazolylacetal-Derivate sind durch die Formel (II) allgemein definiert.

Die Hydroxyethylazolyl-acetal-Derivate der Formel (II) sind bekannt aus der DE—A—3 242 222 und der DE—A 3 242 252 oder lassen sich nach dem dort angegebenen Verfahren erhalten, indem man Oxirane der Formel (VII)

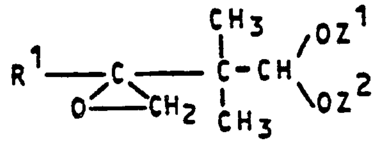

(VII)

in welcher

$R^1$, $Z^1$ und $Z^2$ die oben angegebene Bedeutung haben,

mit Azolen der Formel (VI) in Gegenwart eines inerten organischen Lösungsmittels, wie beispielsweise Alkohole, und gegebenenfalls in Gegenwart einer Base, wie beispielsweise Natriumalkoholat oder Kaliumhydroxid, bei Temperaturen zwischen 60°C und 150°C umsetzt.

Die Oxirane der Formel (VII) sind bekannt aus der DE—A 3 242 252 oder können in allgemein bekannter Art und Weise erhalten werden, indem man Ketone der Formel (VIII),

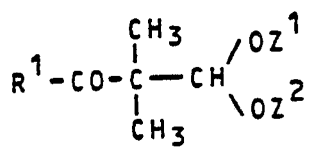

(VIII)

in welcher

$R^1$, $Z^1$ und $Z^2$ die oben angegebene Bedeutung haben, entweder

α) mit Dimethyloxosulfonium-methylid der Formel (IX)

$$\overset{\delta+\ \delta-}{(CH_3)_2SOCH_2} \qquad (IX)$$

in an sich bekannter Weise in Gegenwart eines Verdünnungsmittels, wie beispielsweise Dimethylsulfoxid, bei Temperaturen zwischen 20°C und 80°C umsetzt (vgl. hierzu die Angaben in J.Am.Chem.Soc. *87*, 1363—1364 (1965)), oder

β) mit Trimethylsulfonium-methylsulfat der Formel (X)

$$\left[(CH_3)_3S^{(+)}\right] \quad CH_3SO_4^{(-)} \qquad (X)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0°C bis 60°C, vorzugsweise bei Raumtemperatur, umsetzt (vgl. auch die Angaben in Heterocycles *8*, 397 (1777)).

Die so erhaltenen Oxirane der Formel (VII) können gegebenenfalls ohne Isolierung direkt weiter umgesetzt werden.

Die Ketone der Formel (VIII) sind teilweise bekannt (vgl. z.B. J.Org.Chem. *32*, 404 (1967), DE—A—3 224 130, DE—A—3 224 129 und DE—A—3 242 252). Sie können in bekannter Art und Weise erhalten werden, indem man z.B. 1-(N-Morpholino)-isobuten der Formel (XI)

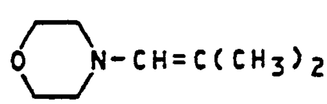

(XI)

mit Chloriden der Formel (XII),

$$R^1\text{—CO—Cl} \qquad (XII)$$

in welcher

$R^1$ die oben angegebene Bedeutung hat,

in Gegenwart eines Lösungsmittels, wie beispielsweise Diethylether, bei Temperaturen zwischen 20°C und

120°C umsetzt und die so erhaltenen Keto-Derivate der Formel (XIII),

$$R^1-CO-\overset{\displaystyle CH_3}{\underset{\displaystyle CH_3}{\overset{|}{\underset{|}{C}}}}-CHO \qquad (XIII)$$

in welcher

$R^1$ die oben angegebene Bedeutung hat,

in üblicher Weise an der Aldehydgruppe derivatisiert, wie z.B. mittels Ethylenglykol in Gegenwart eines inerten organischen Lösungsmittels, wie beispielsweise Toluol, und in Gegenwart einer starken Säure als Katalysator, wie beispielsweise p-Toluolsulfonsäure, bei Temperaturen zwischen 80°C und 110°C.

In manchen Fällen erweist es sich als vorteilhaft, den Rest $R^1$ bzw. Teile davon erst nach der Derivatisierung an der Aldehydgruppe einzuführen (vgl. auch die Herstellungsbeispiele).

Die bei der Durchführung des erfindungsgemäßen Verfahrens (b) als Ausgangsstoffe zu verwendenden Hydroxyethyl-azol-Derivate der Formel (Ia) sind erfindungsgemäße Verbindungen.

Die außerdem für die Durchführung des erfindungsgemäßen Verfahrens (b) als Ausgangsstoffe zu verwendenden Hydroxylamin-Derivate sind durch die Formel (III) allgemein definiert. In dieser Formel steht $R^2$ vorzugsweise für die Bedeutungen, die bereits im Zusammenhang mit der Beschreibung der erfindungsgemäßen Stoffe der Formel (I) vorzugsweise für diesen Substituenten genannt wurden.

Die Hydroxylamin-Derivate der Formel (III) sind allgemein bekannte Verbindungen der organischen Chemie.

Die bei der Durchführung des erfindungsgemäßen Verfahrens (c) als Ausgangsstoffe zu verwendenden Hydroxyethyl-azol-Derivate der Formel (Ib) sind erfindungsgemäße Verbindungen.

Die außerdem für die Durchführung des erfindungsgemäßen Verfahrens (c) als Ausgangsstoffe zu verwendenden Halogenide sind durch die Formel (IV) allgemein definiert. In dieser Formel steht $R^3$ vorzugsweise für die Bedeutungen, die bereits im Zusammenhang mit der Beschreibung der erfindungsgemäßen Stoffe der Formel (I) für $R^2$, außer Wasserstoff, vorzugsweise genannt wurden.

Die Halogenide der Formel (IV) sind allgemein bekannte Verbindungen der organischen Chemie.

Die bei der Durchführung des erfindungsgemäßen Verfahrens (d) als Ausgangsstoffe zu verwendenden Oxirane sind durch die Formel (V) allgemein definiert. In dieser Formel steht $R^1$ vorzugsweise für die Bedeutungen, die bereits im Zusammenhang mit der Beschreibung der erfindungsgemäßen Stoffe der Formel (I) für diesen Substituenten genannt wurden, und $R^3$ steht vorzugsweise für die Bedeutungen, die bereits im Zusammenhang mit der Beschreibung der erfindungsgemäßen Stoffe der Formel (I) für $R^2$, außer Wasserstoff, vorzugsweise genannt wurden.

Die Oxirane der Formel (V) sind noch nicht bekannt; sie können jedoch in allgemein bekannter Art und Weise erhalten werden, indem man Keto-oxim-Derivate der Formel (XIV),

$$R^1-CO-\overset{\displaystyle CH_3}{\underset{\displaystyle CH_3}{\overset{|}{\underset{|}{C}}}}-CH=N-OR^3 \qquad (XIV)$$

in welcher

$R^1$ und $R^2$ die oben angegebene Bedeutung haben,

nach den oben angegebenen Verfahren (α) bzw. (β) epoxidiert.

Die Keto-oxim-Derivate der Formel (XIV) sind bekannt aus der DE—A—3 224 130, der DE—A—3 224 129 und der DE—A—3 242 252. Sie können in bekannter Art und Weise erhalten werden, indem man z.B. 1-(N-Morpholino)-isobuten der Formel (XI) mit Chloriden der Formel (XII) in Gegenwart eines Lösungsmittels, wie beispielsweise Diethylether, bei Temperaturen zwischen 20°C und 120°C umsetzt und die so erhaltenen Keto-Derivate der Formel (XIII) in üblicher Weise an der Aldehydgruppe mittels Hydroxylamin-Derivaten der Formel (III), wie z.B. Methoxyhydroxylamin-hydrochlorid, in Gegenwart eines inerten organischen Lösungsmittels, wie beispielsweise Ethanol und in Gegenwart von Natriumacetat bei Temperaturen zwischen 80°C und 110°C derivatisiert. In manchen Fällen erweist es sich als vorteilhaft, den Rest $R^1$ bzw. Teile davon erst nach der Derivatisierung der Aldehydgruppe einzuführen.

Die außerdem für die Durchführung des erfindungsgemäßen Verfahrens (d) als Ausgangsstoffe zu verwendenden Azole der Formel (VI) sind allgemein bekannte Verbindungen der organischen Chemie.

Das erfindungsgemäße Verfahren (a) wird in Gegenwart eines Gemisches aus Wasser und einem mit Wasser mischbaren, inerten organischen Lösungsmittel durchgeführt. Als organische Lösungsmittel kommen vorzugsweise Alkohole infrage.

Als Säuren kommen für das erfindungsgemäße Verfahren (a) alle üblicherweise verwendbaren anorganischen und organischen Säuren infrage. Hierzu gehören vorzugsweise Salzsäure, Schwefelsäure, p-Toluolsulfonsäure und Essigsäure.

Die Reaktionstemperaturen können bei der Durchführung des erfindungsgemäßen Verfahrens (a) in einem größeren Bereich variiert werden. Im allgemeinen arbeitet man bei Temperaturen zwischen 30°C und 120°C, vorzugsweise bei der Siedetemperatur des verwendeten Lösungsmittels.

Bei der Durchführung des erfindungsgemäßen Verfahrens (a) setzt man vorzugsweise auf 1 Mol der Verbindung der Formel (II) 1 bis 3 Mol, vorzugsweise 2 Mol Säure ein. Die Isolierung der Endprodukte erfolgt in allgemein üblicher Weise.

Als Verdünnungsmittel kommen für das erfindungsgemäße Verfahren (b) vorzugsweise Alkohole und Wasser, bzw. Gemische beider infrage.

Die Reaktionstemperaturen können beim erfindungsgemäßen Verfahren (b) in einem größeren Bereich variiert werden. Im allgemeinen arbeitet man zwischen 20°C und 120°C, vorzugsweise zwischen 50°C und 100°C.

Bei der Durchführung des erfindungsgemäßen Verfahrens (b) setzt man auf 1 Mol der Verbindung der Formel (Ia) vorzugsweise 1 bis 1,5 Mol Hydroxylamin-Derivat der Formel (III) ein. Die Isolierung der Verbindungen der Formel (I) erfolgt nach üblichen Methoden.

Nach einer bevorzugten Ausführungsform des Verfahrens (b) werden die Hydroxylamin-Derivate der Formel (III) in Form ihrer Salze, insbesondere als Hydrochloride, gegebenenfalls in Gegenwart eines Säurebindemittels, wie beispielsweise Natriumacetat, eingesetzt (vgl. auch die Herstellungsbeispiele).

Für die erfindungsgemäße Umsetzung gemäß Verfahren (c) kommen als Verdünnungsmittel inerte organischen Lösungsmittel infrage. Hierzu gehören vorzugsweise Ether, wie Tetrahydrofuran und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Hexamethylphosphorsäure-triamid, Säureamide, wie Dimethylformamid und Sulfoxide, wie Dimethylsulfoxid.

Die erfindungsgemäße Umsetzung gemäß Verfahren (c) wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyltrimethyl-ammoniumhydroxid oder Dibenzyldimethyl-ammoniumhydroxid und Tetraphenylphosphoniumhydroxid oder Methyltriphenylphosphoniumhydroxid.

Die Reaktionstemperaturen können beim Verfahren (c) in einem größeren Bereich variiert werden. Im allgemeinen arbeitet man zwischen 20°C und 150°C, vorzugsweise bei Raumtemperatur. In einzelnen Fällen ist es vorteilhaft, bei der Siedetemperatur des Lösungsmittels, beispielsweise zwischen 60°C und 100°C, zu arbeiten.

Bei der Durchführung des erfindungsgemäßen Verfahrens (c) setzt man auf 1 Mol der Verbindung der Formel (Ib) vorzugsweise 1 bis 3 Mol Halogenid der Formel (IV) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform des Verfahrens (c) wird die erfindungsgemäße Umsetzung in einem Zweiphasensystem, wie beispielsweise wässrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an den Grenzflächen die Alkoholate entstehen und mit den in der organischen Phase befindlichen Halogeniden umgesetzt werden.

Als Verdünnungsmittel kommen für das erfindungsgemäße Verfahren (d) unter den Reaktionsbedingungen inerte organische Lösungsmittel infrage. Hierzu gehören vorzugsweise: Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren (d) alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens (d) in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens (d) setzt man vorzugsweise auf 1 Mol Oxiran der Formel (V) 1 bis 2 Mol Azol und gegebenenfalls 1 bis 2 Mol Base ein. Die Isolierung der Endprodukte erfolgt in allgemein üblicher Weise.

Zur Herstellung von Säureadditions-Salzen der Verbindungen der allgemeinen Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten

inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem. Erfolg zur Bekämpfung von Cochliobolus sativus an Gerste und von Venturia inaequalis am Apfel sowie auch zur Bekämpfung von Mehltau, Septoria nodorum und Pyrenophora teres an Getreide und von Pyricularia oryzae und Pellicularia sesakii an Reis eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfs lösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welcher bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Fräge: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen,

Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## HERSTELLUNGSBEISPIELE

### Beispiel 1

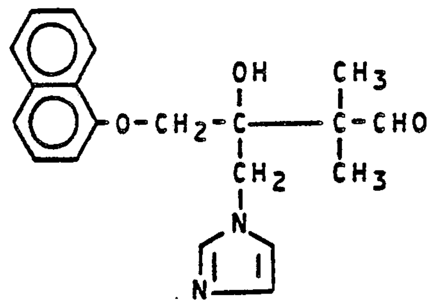

(Verfahren a)

22,7 g (0,059 Mol) 3-(1,3-Dioxolan-2-yl)-2-(imidazol-1-yl-methyl)-3-methyl-1-(naphth-1-yl-oxy)-2-butanol in 150 ml Ethanol und 150 ml Wasser werden mit 15 ml konzentrierter Salzsäure versetzt und 4 Stunden unter Rückfluß erhitzt. Anschließend wird das Reaktionsgemisch in gesättigte, wässrige Natriumhydrogencarbonat-Lösung gegossen und dreimal mit Methylenchlorid extrahiert. Die vereinigten organischen Phasen werden mit Wasser gewaschen, über Natriumsulfat getrocknet und eingeengt. Der Rückstand wird in Ether/Essigester aufgenommen und abgesaugt.

Man erhält 17,0 g (85,3% der Theorie) 2,2-Dimethyl-3-hydroxy-3-(imidazol-1-yl-methyl)-4-(naphth-1-oxy)-butanal vom Schmelzpunkt 147°C.

Herstellung des Ausgangsproduktes

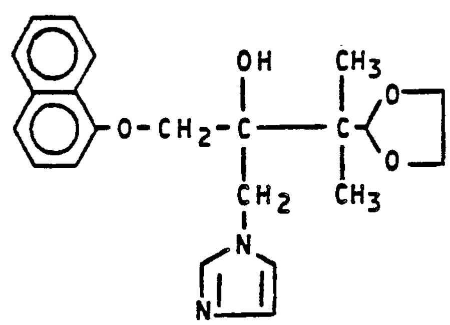

Eine Lösung von 53,5 g (0,17 Mol) 2-[2-(1,3-Dioxolan-2-yl)-prop-2-yl]-2-(naphth-1-yl-oxymethyl)-oxiran, 12,8 g (0,188 Mol) Imidazol und 1,3 g Kaliumhydroxid in 350 ml absolutem Butanol wird 16 Stunden unter Rückfluß erhitzt. Man läßt auf Raumtemperatur abkühlen und versetzt mit 500 ml Methylenchlorid. Das Reaktionsgemisch wird zweimal mit Wasser gewaschen. Die organische Phase wird abgetrennt, über Natriumsulfat getrocknet und eingeengt. Der Rückstand wird mit 350 ml Diisopropylether und Essigester versetzt. Der ausgefallene Niederschlag wird abgesaugt.

Man erhält 38,3 g (59,4% der Theorie) 3-(1,3-Dioxolan-2-yl)-2-(imidazol-1-yl-methyl)-3-methyl-1-(naphth-1-yl-oxy)-2-butanol vom Schmelzpunkt 126°C.

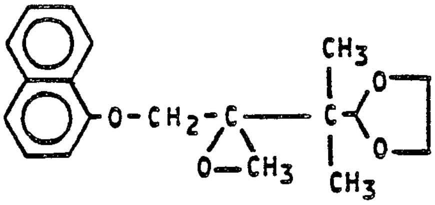

131,1 g (0,596 Mol) Trimethylsulfoxoniumiodid in 120 ml absolutem Dimethylformamid werden portionsweise mit 67,2 g (0,6 Mol) Kalium-tert.-butylat versetzt. Man läßt 6 Stunden bei Raumtemperatur nachrühren und versetzt dann mit einer Lösung von 122 g (0,407 Mol) [2-(1,3-Dioxolan-2-yl)-prop-2-yl]-(naphth-1-yl-oxymethyl)-keton in 550 ml absolutem Tetrahydrofuran. Die Reaktionsmischung wird über Nacht bei Raumtemperatur nachgerührt, eingeengt, in Methylenchlorid aufgenommen, zweimal mit Wasser gewaschen, über Natriumsulfat getrocknet und eingeengt. Der Rückstand wird in Petrolether ausgerührt und abgesaugt.

Man erhält 107 g (83,7% der Theorie) vom Schmelzpunkt 61°C.

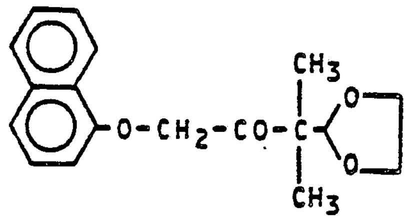

Eine Lösung von 141,5 g (0,735 Mol) 1-Chlor-3-(1,3-dioxolan-2-yl)-3-methyl-2-butanon, 105,9 g (0,835 Mol) 1-Naphthol und 122 g (0,882 Mol) Kaliumcarbonat in 1000 ml absolutem Ethyl-methyl-keton wird 16 Stunden unter Rückfluß erhitzt. Man läßt auf Raumtemperatur abkühlen und filtriert. Das Filtrat wird eingeengt, mit Methylenchlorid versetzt, einmal mit verdünnter Natronlauge und zweimal mit Wasser gewaschen, über Natriumsulfat getrocknet und eingeengt. Der Rückstand wird in Petrolether ausgerührt, abgesaugt und getrocknet.

Man erhält 122,6 g (55,6% der Theorie) [2-(1,3-Dioxolan-2-yl)-prop-2-yl]-(naphth-1-yl-oxymethyl)-keton vom Schmelzpunkt 69°C.

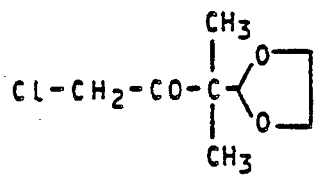

204 g (1,38 Mol) 4-Chlor-2,2-dimethyl-3-keto-butanal werden mit 93 g (1,5 Mol) Ethylenglykol und 0,7 g p-Toluolsulfonsäure in 400 ml Methylenchlorid während 3 Stunden am Wasserabscheider erhitzt. Die organische Phase wird mit 150 ml 5 %-iger Natronlauge und danach mit 400 ml Wasser extrahiert. Das Lösungsmittel wird abdestilliert und der Rückstand im Wasserstrahlvakuum destilliert.

Man erhält 211 g (79,8% der Theorie) 1-Chlor-3-(1,3-dioxolan-2-yl)-3-methyl-butan-2-on vom Siedepunkt 127°C bis 128°C/14 mbar.

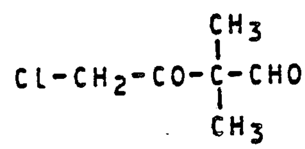

210 g (1,5 Mol) 1-(N-Morpholino)-isobuten werden innerhalb einer Stunde zu 169 g (1,5 Mol) Chloracetylchlorid, gelöst in 350 ml Diethylether, bei 5°C zugetropft. Nach beendeter Zugabe rührt man weitere 3 Stunden unter Rückflußkühlung. Die Lösung wird auf 100 g Eis gegossen, mit wässriger Natriumhydrogencarbonatlösung auf pH 5 gebracht und die Etherphase abgetrennt. Die Wasserphase wird mit 100 ml Diethylether extrahiert, die organischen Phasen werden vereinigt, über Natriumsulfat getrocknet, das Lösungsmittel abdestilliert und der Rückstand im Wasserstrahlvakuum destilliert.

Man erhält 136,4 g (61% der Theorie) 4-Chlor-2,2-dimethyl-3-keto-butanal vom Siedepunkt 95°C bis 98°C/14 mbar.

Beispiel 2

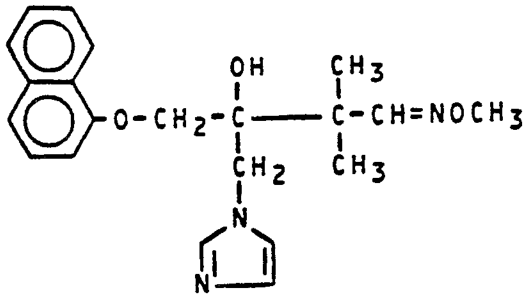

(Verfahren b)

9 g (0,027 Mol) 2,2-Dimethyl-3-hydroxy-3-(imidazol-1-yl-methyl)-4-(naphth-1-oxy)-butanal (Beispiel 1) und 2,2 g (0,027 Mol) O-Methyl-hydroxylamin-hydrochlorid werden in 60 ml Ethanol 16 Stunden unter Rückfluß erhitzt. Anschließend wird das Reaktionsgemisch eingeengt, der Rückstand in Petrolether aufgeschlämmt, abgesaugt und getrocknet.

Man erhält 9 g (90,7% der Theorie) 2-(Imidazol-1-yl-methyl)-3-methoximinomethyl-3-methyl-1-(naphth-1-oxy)-2-butanol vom Schmelzpunkt 177°C bis 178°C.

In analoger Weise und entsprechend den erfindungsgemäßen Verfahrensangaben werden die nachfolgenden Verbindungen der allgemeinen Formel (I) erhalten:

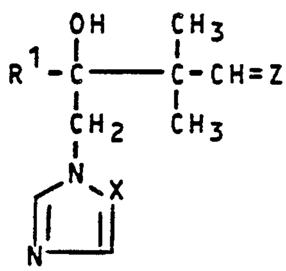

(I)

| Beisp. Nr. | $R^1$ | X | Z | Schmelzpunkt (°C) bzw. spektroskopische Daten |
|---|---|---|---|---|
| 3 | 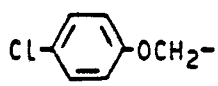 | N | O | 107 |
| 4 | 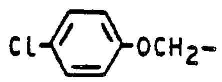 | N | $NOCH_3$ | 144 |
| 5 | 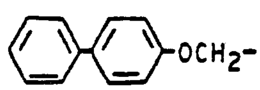 | N | O | Oel/IR$_{CHCl_3}$ : CHO = 1720 $cm^{-1}$ |
| 6 | 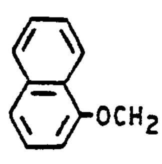 | N | O | Oel/IR$_{CHCl_3}$ : CHO = 1722 $cm^{-1}$ |
| 7 | 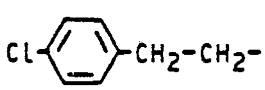 | N | O | 105 |
| 8 | 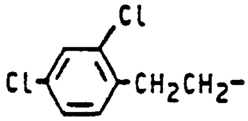 | N | O | 136 |

(Fortsetzung)

| Beisp. Nr. | $R^1$ | X | Z | Schmelzpunkt (°C) bzw. spektroskopische Daten |
|---|---|---|---|---|
| 9 | 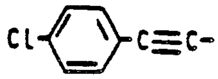 | N | O | 119 |
| 10 | 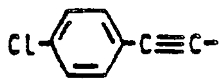 | N | $NOCH_3$ | 121 |
| 11 | 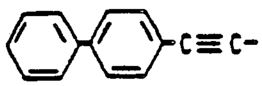 | N | O | 147 |
| 12 | 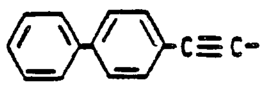 | N | $NOCH_3$ | 151 |
| 13 | 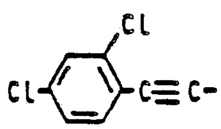 | N | $NOCH_3$ | 93 |
| 14 | 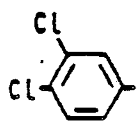 | N | O | 147 - 151 |
| 15 | 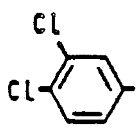 | N | $NOCH_3$ | 96 - 101 |
| 16 | 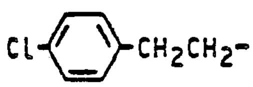 | CH | O | 127 |
| 17 | 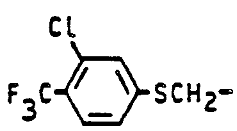 | N | $NOCH_3$ | Oel/$^1H$-$NMR_{CDCl_3}$: = 4,45 ppm für - C$H_2$- 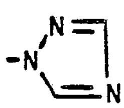 |
| 18 | 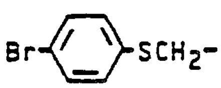 | N | $NOCH_3$ | Oel/$^1H$-$NMR_{CDCl_3}$ : = 4,42 ppm für -C$H_2$- 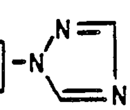 |
| 19 | 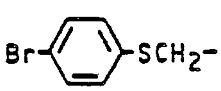 | N | O | Oel/$IR_{CHCl_3}$ : CHO = 1720 $cm^{-1}$ |

(Fortsetzung)

| Beisp. Nr. | $R^1$ | X | Z | Schmelzpunkt (°C) bzw. spektrosk. Daten |
|---|---|---|---|---|
| 20 | 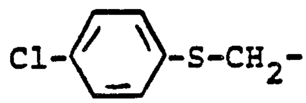 | CH | $NOCH_3$ | Oel/$^1H$-$NMR_{CDCl_3}$: = 4,13 ppm für 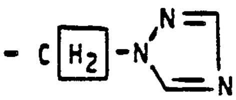 |
| 21 | 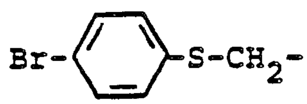 | CH | $NOCH_3$ | Oel/$^1H$-$NMR_{CDCl_3}$: = 4,15 ppm für 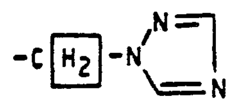 |
| 22 | 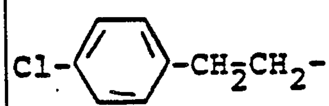 | N | $NOCH_3$ | Oel/$^1H$-$NMR_{CDCl_3}$: = 4,35 ppm für 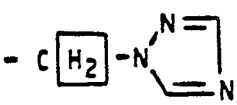 |
| 23 | 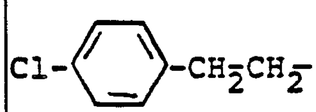 | N | $NOC_4H_9$ | Oel/$^1H$-$NMR_{CDCl_3}$: = 4,35 ppm für 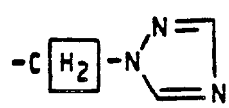 |
| 24 | 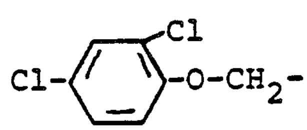 | N | $NOCH_3$ | 119 |
| 25 | 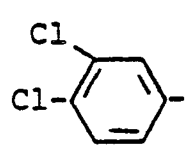 | N | $NOC_2H_5$ | 127-29 |
| 26 | 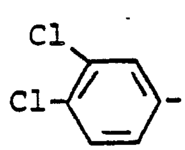 | N | $NOC_4H_9$-n | 138-40 |
| 27 | 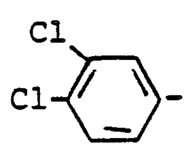 | N | $NOCH_2$-$CH$=$CH_2$ | 140-42 |

(Fortsetzung)

| Beisp. Nr. | $R^1$ | X | Z | Schmelzpunkt (°C) bzw. spektrosk. Daten |
|---|---|---|---|---|
| 28 | 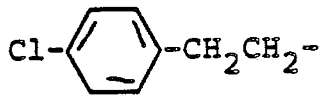 | N | 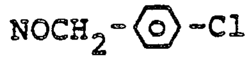 | 125-27 |
| 29 | 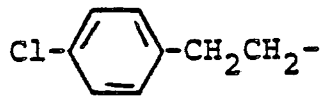 | N | 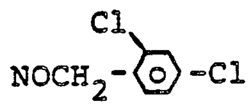 | 97 |
| 30 | 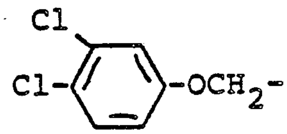 | N | 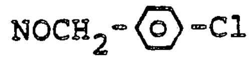 | 89-90 |
| 31 | 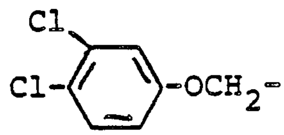 | N | 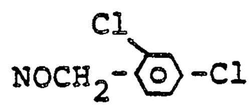 | 72 |
| 32 | 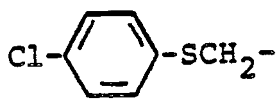 | N | $NOCH_3$ | zähes Öl |
| 33 | 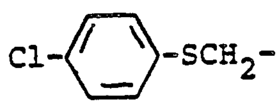 | N | $NOCH_2-CH=CH_2$ | 100-01 |
| 34 | $CH_3$ | N | 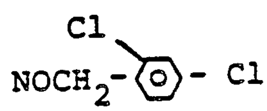 | zähes Öl |
| 35 | 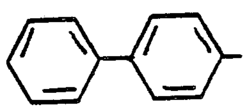 | N | $NOCH_3$ | 55-57 |
| 36 | 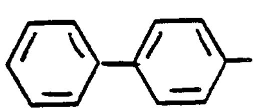 | N | $NOCH_2-CH=CH_2$ | 75 |
| 37 | 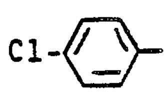 | N | $NOCH_3$ | 96-101 |
| 38 | 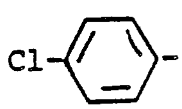 | N | $NOC_3H_7-n$ | 112-14 |

(Fortsetzung)

| Beisp. Nr. | $R^1$ | X | Z | Schmelzpunkt (°C) bzw. spektrosk. Daten |
|---|---|---|---|---|
| 39 | 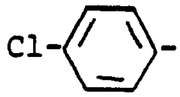 | N | 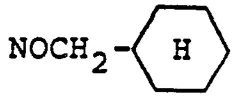 | 130-33 |
| 40 | 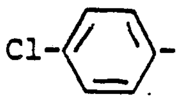 | N | $NOC_4H_9$-n | 124-30 |
| 41 | $C_3H_7$n- | N | 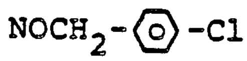 | zähes Öl |
| 42 | $C_3H_7$n- | N | 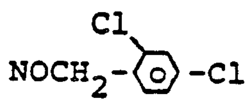 | zähes Öl |
| 43 | $C_2H_5$- | N | 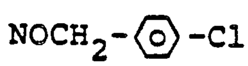 | $n_D^{20}$ = 1,5456 |
| 44 | $C_2H_5$- | N | 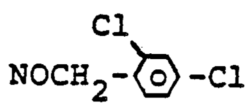 | zähes Öl |
| 45 | $C_4H_9$n- | N | 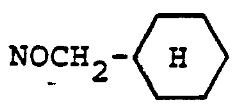 | $n_D^{20}$ = 1,5021 |
| 46 | $C_4H_9$n- | N | 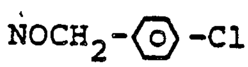 | $n_D^{20}$ = 1,5353 |
| 47 | $C_4H_9$n- | N | 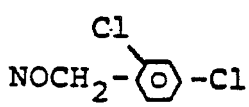 | $n_D^{20}$ = 1,5438 |

Verwendungsbeispiele:

In den nachfolgenden Verwendungsbeispielen werden die nachstehend angegebenen Stoffe als Vergliechsverbindungen eingesetzt:

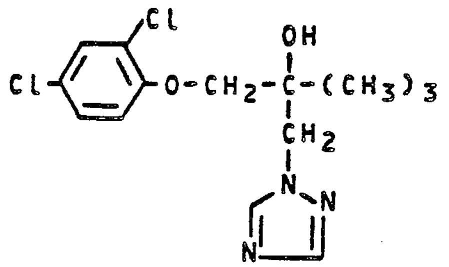

(A)

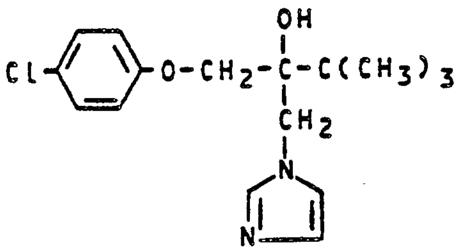

(B)

Beispiel A

Cochliobolus sativus-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewüngschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt.

7 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele: 7, 8, 3 und 4.

Beispiel B

Venturia-Test (Apfel)/protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 16 und 5.

**Patentansprüche**

1. Hydroxyethyl-azol-Derivate der Formel

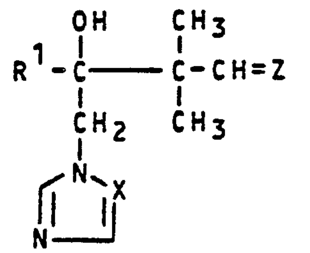

(I)

in welcher

$R^1$ für Alkyl mit 1 bis 6 Kohlenstoffatomen oder für die Gruppierung Ar-Y steht;

Ar für Naphthyl oder Phenyl steht, wobei der Phenylrest einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Nitro, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, den $-CH=NOR^2$-Rest, gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Benzyl und gegebenenfalls durch Halogen und/oder Alkyl mit 1 oder 2 Kohlenstoffatomen substituiertes Benzyloxy,

X für ein Stickstoffatom oder die CH-Gruppe steht;

Y für eine direkte Bindung oder für die Gruppierungen $-CH_2-$, $-CH_2CH_2-$, $-OCH_2-$, $-SCH_2-$, $-CH=CH-$ oder $-C\equiv C-$ steht;

Z für Sauerstoff oder die $NOR^2$-Gruppe steht und

$R_2$ für Wasserstoff, für geradkettiges oder verzweigtes Alkyl mit 1 bis 6 Kohlenstoffatomen, für Alkenyl und Alkinyl mit jeweils 2 bis 6 Kohlenstoffatomen steht oder für Phenylalkyl mit 1 oder 2 Kohlenstoffatomen im Alkylteil steht, wobei der Phenylteil einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit oder 2 Kohlenstoffatomen, Nitro, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Benzyl und gegebenenfalls durch Halogen und/oder Alkyl mit 1 oder 2 Kohlenstoffatomen substituiertes Benzyloxy, oder

$R^2$ für gegebenenfalls einfach bis dreifach, gleich oder verschieden durch Alkyl mit 1 bis 3 Kohlenstoffatomen substituiertes Cycloalkylmethyl mit 5 oder 6 Kohlenstoffatomen im Cycloalkyteil steht, und deren Säureadditions-Salze und Metallsalz-Komplexe.

2. Verbindungen der Formel (I) in Anspruch 1, wobei

$R^1$ für geradkettiges Alkyl mit 1 bis 6 Kohlenstoffatomen oder für die Gruppierung Ar-Y steht;

Ar für Naphthyl oder für gegebenenfalls einfach bis dreifach, gleich oder verschieden durch Fluor, Chlor, Methyl, Trifluormethyl, Trifluormethoxy, Trifluormethylthio, Methoximinomethyl, Ethoximinomethyl, Allyloximinomethyl, sowie jeweils gegebenenfalls durch Chlor und/oder Methyl substituiertes Phenyl, Phenoxy, Benzyl oder Benzyloxy steht,

X für ein Stickstoffatomen oder die CH-Gruppe steht;

Y für eine direkte Bindung oder für Gruppierungen $-CH_2-$, $-CH_2-CH_2-$, $-OCH_2-$, $-SCH_2-$, $-CH=CH-$ oder $-C\equiv C-$ steht;

Z für Sauerstoff oder die $NOR^2$-Gruppe steht; wobei

$R^2$ für Wasserstoff, Methyl, Ethyl, n-Propyl, n-Butyl, Allyl, Propargyl, für gegebenenfalls einfach bis dreifach, gleich oder verschieden durch Fluor, Chlor, Methyl, Trifluormethyl oder Trifluormethoxy substituiertes Benzyl sowie für gegebenenfalls durch Methyl oder Ethyl substituiertes Cyclohexylmethyl steht.

3. Verbindung gemäß Anspruch 1, gekennzeichnet durch die Formel

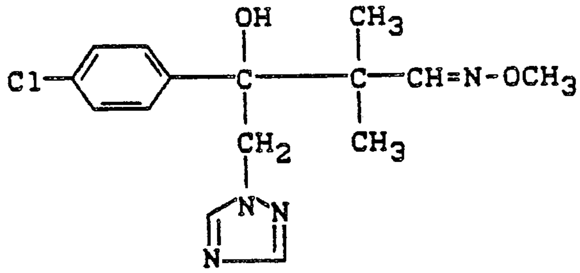

4. Verfahren zur Herstellung on Hydroxyethyl-azol-Derivaten der Formel

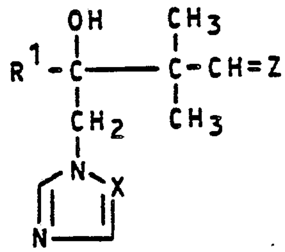

(I)

in welcher

$R^1$ für Alkyl mit 1 bis 6 Kohlenstoffatomen oder für die Gruppierung Ar-Y steht;

Ar für Naphthyl oder Phenyl steht, wobei der Phenylrest einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Nitro, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, den $-CH=NOR^2$-Rest, gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Benzyl und gegebenenfalls durch Halogen und/oder Alkyl mit 1 oder 2 Kohlenstoffatomen substituiertes Benzyloxy,

X für ein Stickstoffatom oder die CH-Gruppe steht;

Y für eine direkte Bindung oder für die Gruppierungen $-CH_2-$, $-CH_2CH_2-$, $-OCH_2-$, $-SCH_2-$, $-CH=CH-$ oder $-C\equiv C-$ steht;

Z für Sauerstoff oder die $NOR^2$-Gruppe steht, und

$R_2$ für Wasserstoff, für geradkettiges oder verzweigtes Alkyl mit 1 bis 6 Kohlenstoffatomen, für Alkenyl und Alkinyl mit jeweils 2 bis 6 Kohlenstoffatomen steht oder für Phenylalkyl mit 1 oder 2 Kohlenstoffatomen im Alkylteil steht, wobei der Phenylteil einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Nitro, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegenbenenfalls durch Halogen und/oder Alkyl mit 1 oder 2 Kohlenstoffatomen substituiertes Benzyl und gegebenenfalls durch Halogen und/oder Alkyl mit 1 oder 2 Kohlenstoffatomen substituiertes Benzyloxy, oder

$R^2$ für gegebenenfalls einfach bis dreifach, gleich oder verschieden durch Alkyl mit 1 bis 3 Kohlenstoffatomen substituiertes Cycloalkylmethyl mit 5 oder 6 Kohlenstoffatomen im Cycloalkyteil steht, sowie von deren Säureadditions-Salzen und Metallsalz-Komplexen, dadurch gekennzeichnet, daß man

a) Hydroxyethylazolyl-acetal-Derivate der Formel

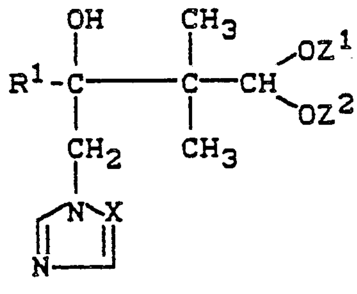

(II)

in welcher

$Z^1$ und $Z^2$ für Alkyl mit 1 bis 4 Kohlenstoffatomen stehen oder gemeinsam mit den Sauerstoffatomen, an die sie gebunden sind, für einen Dioxolan-Ring stehen und

$R^1$ und X die oben angegebene Bedeutung haben,

in Gegenwart eines Gemisches aus Wasser und einem organischen Lösungsmittel und in Gegenwart einer Säure erhitzt; und/oder

b) die nach dem Verfahren a) erhaltenen Hydroxyethyl-azol-Derivat der Formel

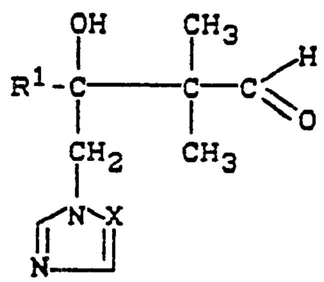

(Ia)

in welcher

$R^1$ und X die oben angegebene Bedeutung haben,

oder die Hydroxyethylazolyl-acetal-Derivate der Formel (II) mit Hydroxylamin-Derivaten der Formel

$$H_2N—O—R^2 \qquad (III)$$

in welcher

$R^2$ die oben engegebene Bedeutung hat, in Gegenwart eines Verdünnungsmittels umsetzt; oder

c) die nach dem Verfahren b) erhaltenen Hydroxyethylazol-Derivate der Formel

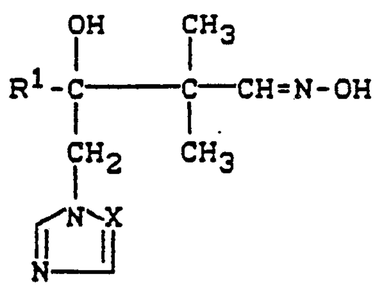

(Ib)

in welcher

$R^1$ und X die oben angegebene Bedeutung haben, mit Halogeniden der Formel

$$Hal\text{-}R^2 \qquad (IV)$$

in welcher

Hal für Chlor, Brom oder Iod steht und

$R^3$ für die Bedeutungen von $R^2$, außer für Wasserstoff, steht, in Gegenwart eines Verdünnungsmittels und gegenbenenfalls in Gegenwart einer Base umsetzt; oder

d) Oxirane der Formel

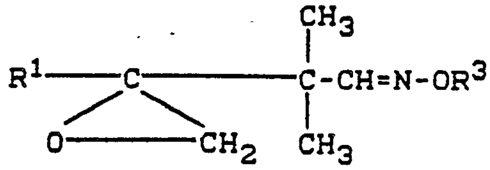

(V)

in welcher

$R^1$ und $R^3$ die oben angegebene Bedeutung haben, mit Azolen der Formel

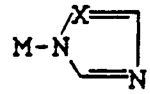

(IV)

in welcher

X die oben angegebene Bedeutung hat un

M für Wasserstoff, Natrium oder Kalium steht,

in Gegenwart eines Verdünnungsmittels und gegebenenfalls in Gegenwart einer Base umsetzt, und gegebenenfalls noch an die so erhaltenen Verbindungen der Formel (I) anschließend eine Säure oder ein Metallsalz addiert.

5. Fungizide Mittel, gekennzeichnet durch einen Gehalt an mindestens einem Hydroxyethyl-azol-Derivat der Formel (I) in Anspruch 1 bzw. an einem Säureadditions-Salz oder Metallsalzkomplex eines Hydroxy-ethyl-azol-Derivates der Formel (I).

6. Verfahren zur Bekämpfung von phytopathogenen Pilzen, dadurch gekennzeichnet, deß man Hydroxyethyl-azol-Derivate der Formel (I) in Anspruch 1 bzw. deren Säureadditions-Salze oder Metallsalz-Komplexe auf diese Pilze oder ihren Lebensraum einwirken läßt.

7. Verwendung von Hydroxyethyl-azol-Derivaten der Formel (I) in Anspruch 1 bzw. von deren Säureadditions-Salzen und Metallsalz-Komplexen als Pflanzenschutzmittel.

8. Verwendung von Hydroxyethyl-azol-Derivaten der Formel (I) in Anspruch 1 bzw. von deren Säureadditions-Salzen und Metallsalz-Komplexen zur Bekämpfung von phytopathogenen Pilzen.

9. Verfahren zur Hellstellung von fungiziden Metteln, dadurch gekennzeichnet, daß man Hydroxyethyl-azol-Derivate der Formel (I) in Anspruch 1 bzw. deren Säureadditions-Salze oder Metallsalz-Komplexe mit Streckmitteln und/oder oberflächenaktiven Mitteln vermischt.

**Revendications**

1. Dérivés de l'hydroxyéthyl-azole de formule

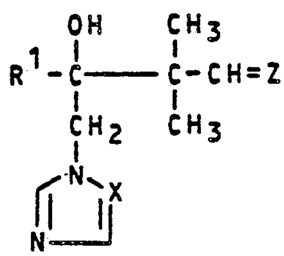

(I)

dans laquelle

$R^1$ représente un groupe alkyle en $C_1$—$C_6$ ou le groupement Ar—Y;

Ar représente un groupe naphtyle ou phényle, le groupe phényle pouvant porter un ou plusieurs substituants indentiques ou différents choisis parmi les halogènes, les groupes alkyle en $C_1$—$C_4$, alcoxy, en $C_1$—$C_2$, alkylthio en $C_1$—$C_2$, nitro, halogénoalkyle contenant 1 ou 2 atomes de carbone et 1 à 5 atomes d'halogènes identiques ou différents, halogénoalcoxy contenant 1 ou 2 atomes de carbone et 1 à 5 atomes d'halogènes identiques ou différents, halogénoalkylthio contenant 1 ou 2 atomes de carbone et 1 à 5 atomes d'halogènes identiques ou différents, le groupe $CH=NOR^2$, les groupes phényle éventuellement substitués par des halogènes et/ou des groupes alkyle en $C_1$—$C_2$, les groupes phénoxy éventuellement substitués par des halogènes et/ou des groupes alkyle en $C_1$—$C_2$, les groupes benzyle éventuellement substitués par des halogènes et/ou des groupes alkyle en $C_1$—$C_2$, et les groupes benzyloxy éventuellement substitués par des halogènes et/ou des groupes alkyle en $C_1$—$C_2$,

X représente un atome d'azote ou le groupe CH,

Y représente une liaison directe ou un groupement $—CH_2—$, $—CH_2CH_2—$, $—OCH_2—$, $—SCH_2—$, $—CH=CH—$ ou $—CH{\equiv}C—$;

Z représente l'oxygène ou le groupe $NOR^2$, et

$R^2$ représente l'hydrogène, un groupe alkyle à chaîne droite ou ramifiée en $C_1$—$C_6$, un groupe alcényle ou alcynyle contenant chacun 2 à 6 atomes de carbone ou un groupe phénylalkyle contenant 1 ou 2 atomes de carbone dans la partie alkyle, la partie phényle pouvant porter un ou plusieurs substituants identiques ou différents choisis parmi les halogènes, les groupes alkyle en $C_1$—$C_4$, alcoxy en $C_1$—$C_2$, alkylthio en $C_1$—$C_2$, nitro, halogènoalkyle contenant 1 ou 2 atomes de carbone et 1 à 5 atomes d'halogènes identiques ou différents, halogénoalcoxy contenant 1 ou 2 atomes de carbone et 1 à 5 atomes d'halogénes identiques ou différents, halogénoalkylthio contenant 1 ou 2 atomes de carbone et 1 à 5 atomes d'halogènes identiques ou différents, les groupes phényle éventuellement substitués par des halogènes et/ou des groupes alkyle en $C_1$—$C_2$, les groupes phénoxy éventuellement substitués par des halogènes et/ou des groupes alkyle en $C_1$—$C_2$, les groupes benzyle éventuellement substitués par des halogènes et/ou des groupes alkyle en $C_1$—$C_2$, et les groupes benzyloxy éventuellement substitués par des halogènes et/ou des groupes alkyle en $C_1$—$C_2$, ou bien

$R^2$ représente un groupe cycloalkylméthyle contenant 5 ou 6 atomes de carbone dans le partie cycloalkyle et portant éventuellement un à trois substituants indentiques ou différents consistant en groupes alkyle en $C_1$—$C_3$,

et leurs sels formés par addition avec des acides et complexes de sels métalliques.

2. Composés de formule I de la revendication 1, dans lesquels

$R^1$ représente un groupe alkyle à chaîne droite en $C_1$—$C_6$ ou le groupement Ar-Y,

Ar représente un groupe naphthyle ou un groupe phényle, phénoxy, benzyle ou benzyloxy portant éventuellement un à trois substituants identiques ou différents, choisis parmi le fluor, le chlore, les groupes méthyle, trifluorométhyle, trifluorométhoxy, trifluorométhylthio, méthoximinométhyle, éthoximinométhyle, allyloximinométhyle, et éventuellement par le chlore et/ou des groupes méthyle,

X représente un atome d'azote ou le groupe CH,

Y représente une liaison directe ou un groupement —$CH_2$—, —$CH_2CH_2$—, —$OCH_2$—, —$SCH_2$—, —CH=CH— ou —CH≡C—,

Z représente l'oxygène ou le groupe $NOR^2$ dans lequel

$R^2$ représente l'hydrogène, un groupe méthyle, éthyle, n-propyle, n-butyle, allyle, propargyle, benzyle portant lui-même éventuellement un à trois substituants identiques ou différents choisis parmi le fluor, le chlore, les groupes méthyle, trifluorométhyle ou trifluorométhoxy, ou cyclohexylméthyle lui-même éventuellement substitué par des groupes méthyle ou éthyle.

3. Composé selon la revendication 1, caractérisé par la formule

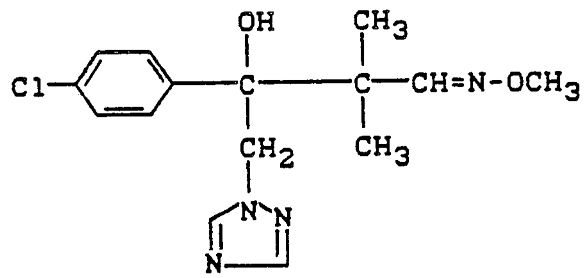

4. Procédé de préparation des derivés de l'hydroxyéthyl-azole de formule

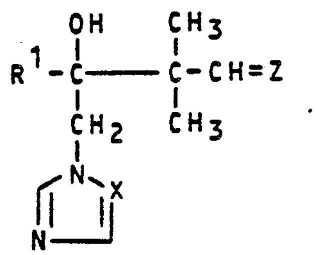

(I)

dans laquelle

$R^1$ représente un groupe alkyle en $C_1$—$C_6$ ou le groupement Ar—Y;

Ar représente un groupe naphtyle ou phényle, le groupe phényle pouvant porter un ou plusieurs substituants indentiques ou différents choisis parmi les halogènes, les groupes alkyle en $C_1$—$C_4$, alcoxy en $C_1$—$C_2$, alkylthio en $C_1$—$C_2$, nitro, halogénoalkyle contenant 1 à 2 atomes de carbone et 1 à 5 atomes d'halogènes identiques ou différents, halogénoalcoxy contenant 1 ou 2 atomes de carbone et 1 à 5 atomes d'halogènes identiques ou différents, halogénoalkylthio contenant 1 ou 2 atomes de carbone et 1 à 5 atomes d'halogènes identiques ou différents, le groupe —$CH=NOR^2$, les groupes phényle éventuellement substitués par des halogènes et/ou des groupes alkyle en $C_1$—$C_2$, les groupes phénoxy éventuellement substitués par des halogènes et/ou des groupes alkyle en $C_1$—$C_2$, les groupes benzyle éventuellement substitués par des halogènes et/ou des groupes alkyle en $C_1$—$C_2$, et les groupes benzyloxy éventuellement substitués par des halogènes et/ou des groupes alkyle en $C_1$—$C_2$,

X représente un atome d'azote ou le groupe CH,

Y représente une liaison directe ou un groupement —$CH_2$—, —$CH_2CH_2$—, —$OCH_2$—, —$SCH_2$—, —CH=CH— ou —C≡C—,

Z représente l'oxygène ou le groupe $NOR^2$, et

$R^2$ représente l'hydrogène, un groupe alkyle à chaîne droite ou ramifiée en $C_1$—$C_6$, un groupe alcényle ou alcynyle contenant chacun 2 à 6 atomes de carbone ou un groupe phénylalkyle contenant 1 ou 2 atomes de carbone dans la partie alkyle, la partie phényle pouvant porter elle-même porter un ou plusieurs substituants identiques ou différents choisis parmi les halogènes, les groupes alkyle en $C_1$—$C_4$, les groupes alcoxy en $C_1$—$C_2$, alkylthio en $C_1$—$C_2$, nitro, halogènoalkyle contenant 1 ou 2 atomes de carbone et 1 à 5 atomes d'halogènes identiques ou différents, halogénoalcoxy contenant 1 ou 2 atomes de carbone et 1 à 5 atomes d'halogénes identiques ou différents, halogénoalkylthio contenant 1 ou 2 atomes de carbone et 1 à 5 atomes d'halogènes identiques ou différents, phényle éventuellement substitués par des halogènes et/ou

des groupes alkyle en $C_1$—$C_2$, phénoxy éventuellement substitués par des halogènes et/ou des groupes alkyle en $C_1$—$C_2$, benzyle éventuellement substitués par des halogènes et/ou des groupes alkyle en $C_1$—$C_2$, et benzyloxy éventuellement substitués par des halogènes et/ou des groupes alkyle en $C_1$—$C_2$, ou bien

$R^2$ représente un groupe cycloalkylméthyle contenant 5 ou 6 atomes de carbone dans le partie cycloalkyle et portant éventuellement un à trois substituants indentiques ou différents choisis parmi les groupes alkyle en $C_1$—$C_3$, et de leurs sels formés par addition avec des acides et complexes de sels métalliques, caractérisé en ce que:

a) on chauffe des dérivés d'hydroxyéthylazolyl-acétals de formule

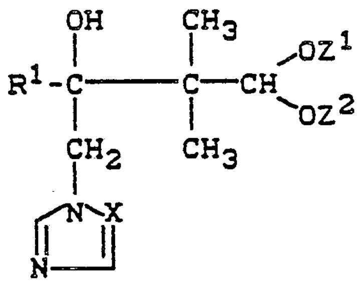

(II)

dans laquelle

$Z^1$ et $Z^2$ représentent des groupes alkyle en $C_1$—$C_4$ ou forment ensemble et avec les atomes d'oxygène auxquels ils sont reliés un cycle dioxolanne, et

$R_1$ et X ont les significations indiquées ci-dessus,

en présence d'un mélange d'eau et d'un solvant organique et en présence d'un acide; et/ou

b) on fait réagir les dérivés de l'hydroxyéthyl-azole obtenus par le procédé a), répondant à la formule

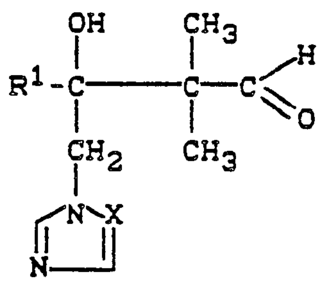

(Ia)

dans laquelle $R^1$ et X ont les significations indiquées ci-dessus, ou bien les dérivés d'hydroxyéthylazolyl-acétals de formule II, avec des dérivés de l'hydroxylamine répondant à la formule

$$H_2N—O—R^2 \qquad (III)$$

dans laquelle $R^2$ a les significant indiquées ci-dessus, en présence d'un diluant; ou bien

c) on fait réagir les dérivés de l'hydroxyéthyl-azole obtenus par le procédé b) et répondant à la formule

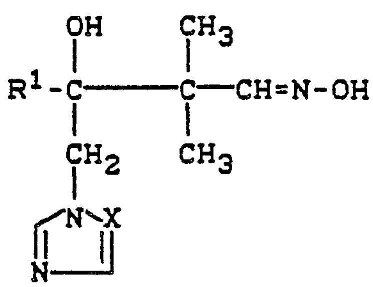

(Ib)

dans laquelle $R^1$ et X ont les significations indiquées ci-dessus, avec des halogénures de formule

$$Hal\text{-}R^3 \qquad (IV)$$

dans laquelle

Hal représente le chlore, le brome ou l'iode, et

$R^3$ a les mêmes significations que $R^2$ à l'exclusion de l'hydrogène, en présence d'un diluant et le cas échéant en présence d'une base; ou bien

d) on fait réagir des oxirannes de formule

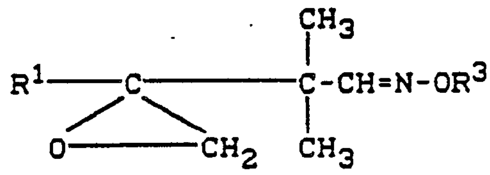

(V)

dans laquelle $R^1$ et $R^3$ on les significations indiquées ci-dessus, avec des azole de formule

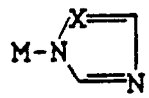

(IV)

dans laquelle

X a les significations indiquées ci-dessus, et

M représente l'hydrogène, le sodium ou le potassium, en présence d'un diluant et le cas échéant en présence d'une base, et éventuellement on fixe ensuite les composés de formule I ainsi obtenus; par addition, sur an acide ou un sel métallique.

5. Produits fongicides caractérisé en ce que qu'ils contiennent au moins un dérivé de l'hydroxyéthyl-azole de formule I de la revendication 1 ou un sel formé par addition avec un acide ou complexe de sel métallique d'un dérivé de l'hydroxyéthyl-azole de formule I.

6. Procédé pour combattre les mycétes phytopathogènes, caractérisé en ce que l'on fait agir des dérivés de l'hydroxyéthyl-azole de formule I de la revendication 1 ou leurs sels formés par addition avec des acides ou complexes de sels métalliques sur ces mycètes ou leur habitat.

7. Utilisation des dérivés de l'hydroxyéthyl-azole de formule I de la revendication 1 ou de leurs sels formés par addition avec des acides et complexes de sels métalliques en tant qui produits phytosanitaires.

8. Utilisation des dérivés de l'hydroxyéthyl-azole de formule I de la revendication 1 ou de leurs sels formés par addition avec des acides et complexes de sels métalliques dans la lutte contre les mycétes phytopathogènes.

9. Procédé de préparation de produits, fongicides, caractérisé en ce que l'on mélange des dérivés de l'hydroxyéthyl-azole de formule I de la revendication 1 ou leurs sels formés par addition avec des acides ou complexes de sels métalliques avec des diluants et/ou des agents tensioactifs.

**Claims**

1. Hydroxyethyl-azole derivatives of the formula

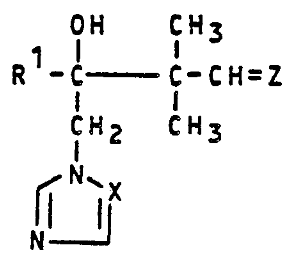

(I)

in which

$R^1$ represents alkyl having 1 to 6 carbon atoms or the group Ar—Y;

Ar represents naphthyl or phenyl, it being possible for the phenyl part to be monosubstituted or polysubstituted by identical or different substituents, substituents which may be mentioned being: halogen, alkyl with 1 to 4 carbon atoms, alkoxy with 1 or 2 carbon atoms, alkylthio with 1 or 2 carbon atoms, nitro, halogenoalkyl with 1 or 2 carbon atoms and 1 to 5 identical or different halogen atoms, halogenoalkoxy with 1 or 2 carbon atoms and 1 to 5 identical or different halogen atoms, halogenoalkylthio with 1 or 2 carbon atoms and 1 to 5 identical or different halogen atoms, the $—CH=NOR^2$ radical, phenyl which is optionally substituted by halogen and/or alkyl with 1 or 2 carbon atoms, phenoxy which is optionally substituted by halogen and/or alkyl with 1 or 2 carbon atoms, benzyl which is optionally substituted by halogen and/or alkyl with 1 or 2 carbon atoms, and benzyloxy which is optionally substituted by halogen and/or alkyl with 1 or 2 carbon atoms,

X represents a nitrogen atom or the CH group;

Y represents a direct bond or the group $-CH_2-$, $-CH_2-CH_2-$, $-OCH_2-$, $-SCH_2-$, $-CH=CH-$ or $-C{\equiv}C-$;

Z represents oxygen or the $NOR^2$ group and

$R^2$ represents hydrogen, or represents straight-chain or branched alkyl with 1 to 6 carbon atoms, or represents alkenyl and alkinyl with in each case 2 to 6 carbon atoms, or represents phenylalkyl with 1 or 2 carbon atoms in the alkyl part, it being possible for the phenyl part to be monosubstituted or polysubstituted by identical or different substituents, substituents which may be mentioned being: halogen, alkyl with to to 4 carbon atoms, alkoxy with 1 or 2 carbon atoms, alkylthio with 1 or 2 carbon atoms, nitro, halogenoalkyl with 1 or 2 carbon atoms and 1 to 5 identical or different halogen atoms, halogenoalkoxy with 1 or 2 carbon atoms and 1 to 5 identical or different halogen atoms, halogenoalkylthio with 1 or 2 carbon atoms and 1 to 5 identical or different halogen atoms, phenyl which is optionally substituted by halogen and/or alkyl with 1 or 2 carbon atoms, phenoxy which is optionally substituted by halogen and/or alkyl with 1 or 2 carbon atoms, benzyl which is optionally substituted by halogen and/or alkyl with 1 or 2 carbon atoms, and benzyloxy which is optionally substituted by halogen and/or alkyl with 1 or 2 carbon atoms, or

$R^2$ represents cycloalkylmethyl which has 5 or 6 carbon atoms in the cycloalkyl part and which is optionally monosubstituted to trisubstituted by identical or different alkyl substituents with 1 to 3 carbon atoms, and their acid addition salts and metal salt complexes.

2. Compounds of the formula (I) in Claim 1, where

$R^1$ represents straight-chain alkyl with 1 to 6 carbon atoms or the group AR—Y—;

Ar represents naphthyl or phenyl which is optionally monosubstituted to trisubstituted by identical or different substituents, substituents which may be mentioned being: fluorine, chlorine, methyl, trifluoromethyl, trifluoromethoxy, trifluoromethylthio, methoxyiminoethyl, ethoximinomethyl or allyloximinomethyl, or represents phenyl, phenoxy, benzyl or benzyloxy, each of which is optionally substituted by chlorine and/or methyl,

X represents a nitrogen atom or the CH group;

Y represents a direct bond or the groups $-CH_2-$, $-CH_2-CH_2-$, $-OCH_2-$, $-SCH_2-$, $-CH=CH-$ or $-C{\equiv}C-$;

Z represents oxygen or the $NOR^2$ group; where

$R^2$ represents hydrogen, methyl, ethyl, n-propyl, n-butyl, allyl or propargyl, or represents benzyl which is optionally monosubstituted to trisubstituted by identical or different substituents, substituents which may be mentioned being: flourine, chlorine, methyl, trifluoromethyl or trifluoromethoxy, or represents cyclohexylmethyl which is optionally substituted by methyl or ethyl.

3. Compound according to Claim 1, characterized by the formula

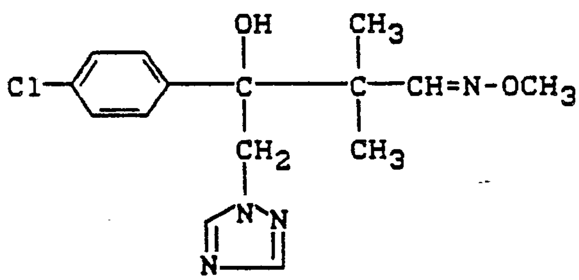

4. Process for the preparation of hydroxyethyl-azole derivatives of the formula

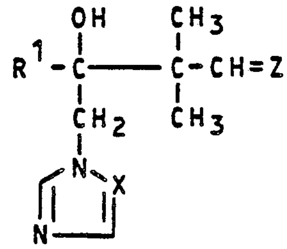

(I)

in which

$R^1$ represents alkyl having 1 to 6 carbon atoms or the group Ar-Y;

Ar represents naphthyl or phenyl, it being possible for the phenyl part to be monosubstituted or polysubstituted by identical or different substituents, substituents which may be mentioned being: halogen, alkyl with 1 to 4 carbon atoms, alkoxy with 1 or 2 carbon atoms, alkylthio with 1 or 2 carbon atoms, nitro, halogenoalkyl with 1 or 2 carbon atoms and 1 to 5 identical or different halogen atoms,

halogenoalkoxy with 1 or 2 carbon atoms and 1 to 5 identical or different halogen atoms, halogenoalkylthio with 1 or 2 carbon atoms and 1 to 5 identical or different halogen atoms, the —CH=NOR$^2$ radical, phenyl which is optionally substituted by halogen and/or alkyl with 1 or 2 carbon atoms, phenoxy which is optionally substituted by halogen and/or alkyl with 1 or 2 carbon atoms, benzyl which is optionally substituted by halogen and/or alkyl with 1 or 2 carbon atoms, and benzyloxy which is optionally substituted by halogen and/or alkyl with 1 or 2 carbon atoms,

X represents a nitrogen atom or the CH group;

Y represents a direct bond or the group —$CH_2$—, —$CH_2$—$CH_2$—, —$OCH_2$—, —$SCH_2$—, —CH=CH— or —C≡C—;

Z represents oxygen or the NOR$^2$ group and

R$^2$ represents hydrogen, or represents straight-chain or branched alky with 1 to 6 carbon atoms, or represents alkenyl and alkinyl with in each case 2 to 6 carbon atoms, or represents phenylalkyl with 1 or 2 carbon atoms in the alkyl part, it being possible for the phenyl part to be monosubstituted or polysubstituted by identical or different substituents, substituents which may be mentioned being: halogen, alkyl with 1 to 4 carbon atoms, alkoxy with 1 or 2 carbon atoms, alkylthio with 1 or 2 carbon atoms, nitro, halogenoalkyl with 1 or 2 carbon atoms and 1 to 5 identical or different halogen atoms, halogenoalkoxy with 1 or 2 carbon atoms and 1 to 5 identical or different halogen atoms, halogenoalkylthio with 1 or 2 carbon atoms and 1 to 5 identical or different halogen atoms, phenyl which is optionally substituted by halogen and/or alkyl with 1 or 2 carbon atoms, phenoxy which is optionally substituted by halogen and/or alkyl with 1 or 2 carbon atoms, benzyl which is optionally substituted by halogen and/or alkyl with 1 or 2 carbon atoms, and benzyloxy which is optionally substituted by halogen and/or alkyl with 1 or 2 carbon atoms, or

R$^2$ represents cycloalkylmethyl which has 5 or 6 carbon atoms in the cycloalkyl part and which is optionally monosubstituted to trisubstituted by identical or different alkyl substituents with 1 to 3 carbon atoms, and of their acid addition salts and metal salt complexes, charcterized in that

a) hydroxyethylazolyl-acetal derivatives of the formula

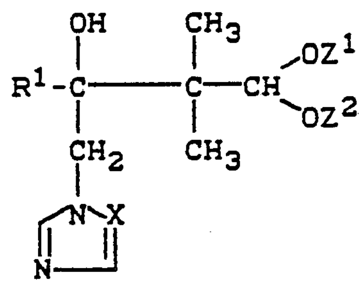

(II)

in which

Z$^1$ and Z$^2$ represent alkyl with 1 to 4 carbon atoms, or together with the oxygen atoms to which they are bonded represent a dioxolane ring, and

R$^1$ and X have the abovementioned meaning, are heated in the presence of a mixture of water and an organic solvent and in the presence of an acid; and/or

b) the hydroxyethyl-azole derivatives, obtained by process (a), of the formula

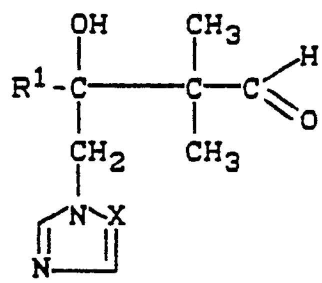

(Ia)

in which

R$^1$ and X have the abovementioned meaning, or the hydroxyethylazolyl-acetal derivatives of the formula (II) are reacted with hydroxylamine derivatives of the formula

$$H_2N—O—R^2 \qquad (III)$$

in which

R$^2$ has the abovementioned meaning, in the presence of a diluent; or

c) the hydroxyethyl-azole derivatives, obtained by process b), of the formula

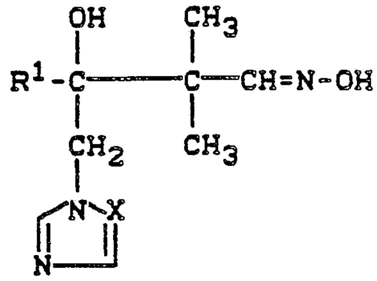

(Ib)

in which

$R^1$ and X have the abovementioned meaning, are reacted with halides of the formula

Hal-$R^3$ (IV)

in which

Hal represents chlorine, bromine or iodine and

$R^3$ represents the meanings of $R^2$, with the exception of hydrogen, in the presence of a diluent and if appropriate in the presence of a base; or

d) oxiranes of the formula

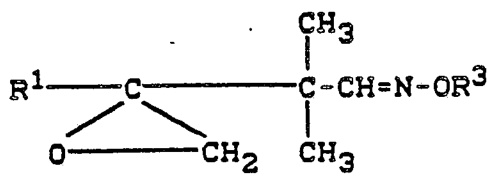

(V)

in which

$R^1$ and $R^3$ have the abovementioned meaning, are reacted with azoles of the formula

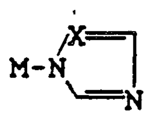

(IV)

in which

X has the abovementioned meaning and

M represents hydrogen, sodium or potassium, in the presence of a diluent and if appropriate in the presence of a base, and, if appropriate, an acid or a metal salt are then additionally added on to the compounds of the formula (I).

5. Fungicidal agents, characterized in that they contain at least one hydroxyethyl-azole derivative of the formula (I) in Claim 1, or an acid addition salt or metal salt complex of a hydroxyethyl-azole derivative of the formula (I).

6. Method of combating phytopathogenic fungi, characterized in that hydroxyethyl-azole derivatives of the formula (I) in Claim 1, or their acid addition salt or metal salt complexes, are allowed to act on these fungi or their environment.

7. Use of hydroxyethyl-azole derivatives of the formula (I) in Claim 1, or of their acid addition salts and metal complexes, as plant protection agents.

8. Use of hydroxyethyl-azole derivatives of the formula (I) in Claim 1, or their acid addition salts and metal complexes, for combating phytopathogenic fungi.

9. Process for the preparation of fungicidal agents, characterized in that hydroxyethyl-azole derivatives of the formula (I) in Claim 1, or their acid addition salts or metal salt complexes, are mixed with extenders and/or surface-active agents.